(19) Europäisches Patentamt
European Patent Office
Office européen des brevets

(11) Publication number: **0 441 469 A1**

# (12) EUROPEAN PATENT APPLICATION

(21) Application number: **91300007.1**

(22) Date of filing: **02.01.91**

(51) Int. Cl.5: **G01N 33/543**, C12Q 1/66

(30) Priority: **19.01.90 US 467649**

(43) Date of publication of application:
**14.08.91 Bulletin 91/33**

(84) Designated Contracting States:
**AT BE CH DE DK ES FR GB GR IT LI LU NL SE**

(71) Applicant: **PROMEGA CORPORATION**
**2800 Woods Hollow Road**
**Madison, WI 53711-5399(US)**

(72) Inventor: **Sivesind, Terry M.**
**2800 Woods Hollow Road**
**Madison, Wisconsin 53711-5305(US)**

Inventor: **Dimond, Randall L.**
**4409 Travis Terrace**
**Madison, Wisconsin 53711(US)**
Inventor: **Schumm, James W.**
**5843 Timber Ridge Trail**
**Madison, Wisconsin 53711(US)**
Inventor: **Pahuski, Edward E.**
**514 Maunesha Drive**
**Marshall, Wisconsin 53559(US)**

(74) Representative: **Ellis-Jones, Patrick George Armine**
**J.A. KEMP & CO. 14 South Square Gray's Inn**
**London WC1R 5LX(GB)**

## (54) Immunospecific and bioluminescent assay of cellular ATP.

(57) The detection of living organisms through the use of a bioluminescent assay for a living cell detection metabolite such as ATP with an antibody-like mediated cell capture onto a solid phase is described. The assay utilizes detection methods after immunospecific cell capture to a solid phase. The method involves cell capture, removal of non-specific or other contaminating materials, cell lysis and detection of the detection metabolite utilizing a light producing enzyme such as the firefly-luciferase enzyme technique. The invention is also directed to kits for performing such detections.

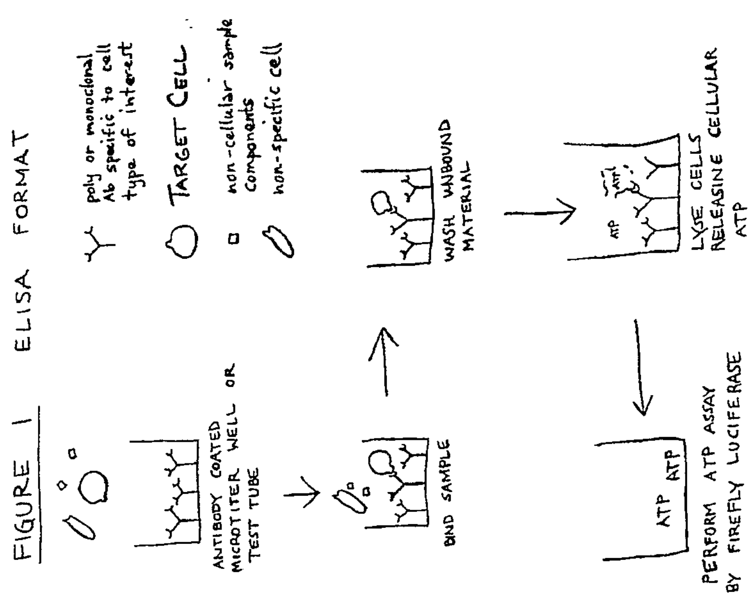


EP 0 441 469 A1

# IMMUNOSPECIFIC AND BIOLUMINESCENT ASSAY OF CELLULAR ATP

## FIELD OF THE INVENTION

The present invention is generally directed to the detection of living organisms in a fluid, especially in an aqueous physiological fluid. The present invention is specifically directed to the detection of the presence and concentration of specified living organisms in a physiological fluid sample through the use of a bioluminescent assay of certain detection metabolites in the cells of living organisms captured onto a solid phase by a cell capture system.

## BACKGROUND OF THE INVENTION

Procedures for the rapid, positive identification of specific organisms in samples, such as food, beverages, cosmetic preparations or body fluid samples, are important, particularly when the determination relates to a matter of health concern. In such cases a determination is required within a short time, generally a few hours.

The prior art has relied on standard microbiological techniques for the determination of the presence and concentration of living organisms in a sample. These techniques are based on the biochemical or growth characteristics of the organism, and often involve single or multiple enrichment steps and selective culture methods. Reference is made to Flowers, R.S. (March, 1985) Food Technology, pp. 103-108, for a description of this technique. However, these procedures are labor intensive, costly, take extended periods of time before results are available, and lack the necessary specificity. (Mattingly, J.A., et al., March 1985, Food Technology, pp. 90-94).

A second technique, known as the fluorescent-antibody (FA) technique, was one of the first immunological methods utilized for the detection of Salmonella. (Thomason, et al., 1957, J. Bacteriol., Vol. 74, p. 525). Although the FA technique is more rapid than standard microbiological techniques, it is time consuming, taking up to 50 hours to conduct. There are also problems with false-positive reactions. As such, all positive results must be reconfirmed using conventional microbiological or culture testing. The process is also expensive, requiring special fluorescent microscopic equipment. Additionally, personnel must be well trained to recognize the positive fluorescent reactions, and fatigue is often a problem in reading large numbers of samples by the FA technique (Mattingly, et al., supra.; and Silliker, et al., 1982, Food Technology, Vol. 36, p. 65).

One of the more recent techniques for cell detection includes immunological tests such as ELISA (Enzyme Linked Immunosorbant Assay) as described in D'Aoust, J.Y., et al. (1986), J. Food Science, Vol. 51, pp. 484-488; and Swaminatham, B., et al. (1985), Food Technology, pp. 83-88. In this method, cells are captured on a solid phase immunospecifically, and a second signaling antibody is used to complete a sandwich. While this method is an improvement over the FA technique in that it requires less time, is less expensive to perform, and does not require technicians having the skills necessary to perform an FA technique, the immunological assay still lacks the sensitivity necessary to do one day testing.

Nucleic acid based probe assay systems have also been developed that are sequence specific for the organisms of interest, as described in Fitts, R. (March 1985), Food Technology, pp. 95-100. Reference is also made to U.S. Patent 4,689,295 to Tabor et al., which is directed to a method of detecting the presence of Salmonella utilizing a DNA probe. These procedures include cell extraction, hybridization and detection steps. However, the nucleic acid based probe assay also lacks adequate sensitivity to allow one day testing.

Sensitive assays for the determination of the presence of adenosine triphosphate (ATP), a component of all living cells, have been described in Lundin, A. (1982), Luminescent Assays: Prospectives in Endocrinology and Chemical Chemistry, Ed. Serio and Pazzagli, Raven Press, New York, and DeLuca M. (1976) Advances in Enzymology, Ed. A. Meister, Vol. 44, pp. 37-68. Bacterial ATP levels may be used as a measure of bacterial cell numbers. This is an important consideration in fields of medical microbiology, food and dairy microbiology and environmental microbiology and the cosmetics industry.

ATP can also be measured using a well-known firefly bioluminescence assay, which is described in U.S. Patent 3,745,090 to Chappelle, et al. The bioluminescent assay is specific for ATP, thereby making it a suitable indicator of the presence of various life forms. The bioluminescent assay is performed by causing the release of ATP from living cells present in a concentrated sample aliquot, reacting the ATP with an enzyme resulting in a light reaction relating to the amount of ATP present, measuring the light, and relating the light reading to the concentration of bacteria present. The test is basically accomplished by reacting ATP with the firefly-luciferase enzyme and luciferin in the presence of a divalent metal ion such as

magnesium or manganese. Luciferase is an enzyme which catalyzes the reaction of luciferin with ATP preferably in the presence of $Mg^{++}$, with light emission being one of the results of the reaction.

Using this system, a number of applications have been described, including immunoassays (Carrico, R., et al., 1976, Anal. Biochem., Vol. 76, pp. 95-110); drug susceptibility tests (Proceeding of the 2nd Bi-Annual ATP Methodology Symposium, 1977 ed. G. Borun, pp. 219-235, SAI Tech. Co., San Diego, CA); enzymology (De Luca, M., 1978, Methods in Enzymology, Vol. LVII, pp. 56-65); bioassays (Nilsson, L., 1979, Antimicro. Agents Chemo., Vol. 14, pp. 812-816); bacteriuria analysis (Thore, A., et al., 1975, J. Clin. Microbiol., Vol. 1, pp. 1-8); and microbial detection in raw milk (Bossuyt, R., 1982, Neth. Milk Dairy J., Vol. 36, pp. 355-364.)

Reference is also made to PCT International Publication No. WO 89/02929 to Lundin, et al., which is directed to a diagnostic means for the luminometric assay of ATP for use in clinical microbiology, veterinary medicine and the food industry. The assay is based on the firefly-luciferase reaction resulting in light emission proportional to ATP concentration in a sample. U.S. Patent 3,971,703 to Picciolo, et al. is directed to a method of detecting and counting bacteria in a sample fluid by measuring bacterial ATP concentration in the sample with a luciferase-luciferin mixture. U.S. Patent 4,385,113 to Chappelle, et al. is directed to the determination of bacteria in water by adding nitric acid to a water sample with concentrated bacteria to rupture the bacterial cells, diluting the sample, mixing the sample with a luciferase-luciferin mixture and measuring the resulting light output of the bioluminescent reaction. U.S. Patent 4,264,727 to Kolehmainen, et al. is directed to a method for assaying digoxin, a drug used to prevent thrombosis, in blood serum. The method is based on the utilization of sodium-potassium specific ATPase as a receptor for digoxin.

While all of the techniques described above may be used to perform specific assays, there is still a need for a rapid and sensitive assay for the detection of the presence and concentration of specific cell types in a sample.

## SUMMARY OF THE INVENTION

It is therefore an object of the present invention to provide a method and a kit for the detection of specific cell types in a sample.

It is additionally an object of the present invention to provide a rapid and sensitive assay technique for analyzing samples, particularly food and beverage samples, that may be contaminated with an unwanted microorganism such as Salmonella.

It is further an object of the present invention to provide a rapid and sensitive technique for analyzing biological specimens, such as body fluids and tissues, for the presence of specific cell types. The target organisms may be either prokaryotic or eukaryotic cells.

It is still another object of the present invention to provide a rapid and sensitive assay system and kit for determining the concentration of a specific cell type in a sample.

These and other objects are addressed by the present invention, which is directed to a process for determining the presence of an antigenic cell substance in a fluid sample, comprising contacting the sample with a cell binding molecule designed to bind to the antigenic cell substance, the cell binding molecules being bound to a solid carrier which is insoluble in the fluid sample. This forms a complex between the cell binding molecule and the antigen. The solid carrier is separated from the fluid sample, and the cell walls of the antigenic cells are lysed to release one of several known cellular detection metabolites. A light producing enzyme is added to the treated sample and the presence of the detection metabolites is detected.

The present invention is further directed to a process for determining the presence of an antigenic cell substance in a fluid sample comprising contacting the sample with an antibody to the antigenic cell substance. The antibody is bound to a solid carrier which is insoluble in the fluid sample. A complex is formed between the antibody and the antigen. The antibody-antigen complex is captured onto the solid carrier. The solid carrier is then separated from the fluid sample, and the cell walls of the antigenic cells are lysed to release cellular ATP. A firefly-luciferase reagent is added to the treated sample and the presence of ATP is detected by the firefly-luciferase reaction.

The present invention is also directed to a process for determining the concentration of an antigenic cell substance in a fluid sample which includes, along with the steps described above, measuring the emitted light from the treated sample, calculating the concentration of the detection metabolite based on the emitted light and calculating the antigenic cell concentration in the sample.

The present invention is also directed to a diagnostic kit for the detection of an antigenic cell substance in a sample.

The present invention is specifically directed to utilizing an ATP detection method with the firefly-luciferase system after the immunospecific capture of the targeted cell to a solid phase carrier. A specific target cell type can be detected from a mixture of other cell types or sample components. This assay and testing method is also applicable to eukaryotic cell types. These methods can utilize either polyclonal or monoclonal antibodies to capture target cells.

The assay provides improved sensitivity and specificity over other cell detection methods by incorporating the advantages of antibody specificity in the assay system.

Without wishing to be bound to one particular theory, it is suggested that the reasoning for the improved sensitivity and specificity is as follows: the limit of sensitivity of an ELISA technique for Salmonella is reported to be $10^5$ cells per ml. (Swaminathem, et al., supra). It is estimated that the amount of ATP in a single bacterium is on the order of $40 \times 10^{-18}$M (Thore, et al., supra). Given that the sensitivity of commercial ATP assays on available instrumentation is on the order of $2 \times 10^{-16}$M, a theoretical estimate of the maximum sensitivity of the ATP assay is 50 bacterial cells. Thus, the present invention still offers a significant improvement in sensitivity over other known methods because of a lower detection limit.

Another advantage of the present invention is that the assay system measures only live cells. Thus, the assay system has enhanced sensitivity to living organisms. This is because most detection metabolites, specifically ATP, are rapidly broken down in dead cells. This has an advantage especially in food microbiology testing as live cells are generally the cause of potential problems in a food or beverage sample.

The present invention has many applications, including novel methods and kits for the detection of the presence and concentration of specific cell types in a sample. Further, the present invention is useful in the analysis of food or beverage samples that may be contaminated with microorganisms such as Salmonella. Additionally, the assay system provides a method for the analysis of biological specimens such as body fluids and tissues for the presence of specific cell types, either prokaryotic or eukaryotic. The assay system of the present invention is distinct from other methods and is amenable to a wide range of formats in order to study various biological samples. The assay system is directed to all types of assay systems whereby a specific cell type is to be detected from a mixture of other cell types in the sample. This would include such samples as biological fluids, including body fluids, foods and liquids, and all various manipulations of foods and liquids necessary in general testing of consumable products. Additionally, this would include samples from bacteriological enrichment steps of cells of any source including consumable items, such as pre-enrichment or selective media growth. The capture of these cells is mediated by either polyclonal or monoclonal antibodies, or other cell binding molecules.

Further objects, features and advantages of the present invention will be apparent from the following detailed description when taken in conjunction with the accompanying drawings.

## BRIEF DESCRIPTION OF THE DRAWINGS

In the drawings,

Figure 1 illustrates in schematic form one embodiment of the present invention utilizing the ELISA format.

Figure 2 illustrates in schematic form a second embodiment of the present invention utilizing the dipstick format.

Figure 3 illustrates in schematic form a third embodiment of the present invention utilizing a magnetic solid phase.

Figure 4 illustrates in schematic form a fourth embodiment of the present invention utilizing a magnetic solid phase using biotin/streptavidin.

Figure 5 illustrates in schematic form a fifth embodiment of the present invention utilizing a magnetic solid phase with protein A.

Figure 6 is a graph illustrating the results of Example 1.

Figure 7 is a graph illustrating the results of Example 2.

## DETAILED DESCRIPTION OF THE INVENTION

In general, the present invention is directed to an immunological assay technique that utilizes the cell binding specificity of an ELISA-type test, but is more sensitive due to the detection of certain detection metabolites, such as ATP, in captured cells. Cells are captured immunospecifically to a solid carrier and are then detected using a light producing enzyme for the detection of the detection metabolite. The present invention is further directed to all assay systems whereby a specific cell type is to be detected from a mixture of other cell types or sample components. This would include such samples as foods and liquids

and all various manipulations of foods and liquids necessary in general testing of consumable products.

The present invention describes the use of a specific cell capture process coupled with ATP detection as a method of cell detection.

## Definitions

As used herein, the term "antigen" or "antigenic cell substance," "antigenic cell," "antigenic substance" or "target cell" is intended to mean any prokaryotic or eukaryotic cellular organism which is amenable to capture with cell binding molecules. In addition, any macromolecular entity containing ATP or another detection metabolite, which can be captured with a cell binding molecule, is also a target cell within the scope of this invention.

The term "bacteria" is meant to include single-celled prokaryotes. It is also within the scope of the present invention to interchange the term "bacteria" with the terms "antigen" and "antigenic cell substance."

The term "eukaryotic cell" is intended to denote higher organisms in which the genetic material is enclosed by a nucleus.

The term "cell binding molecule" is meant to include all types of molecules which will effectively bind an antigenic cell substance to a solid phase carrier. Cell binding molecules include antibodies and molecules engineered to look and work like antibodies for the purpose of forming complexes similar to antibody-antigen complexes.

The term "antibody" or "specific binding protein" denotes any macromolecule that may, by specific interaction with a target cell component, bind the cell of interest in a capture or attachment technique.

The term "detection metabolite" is meant to include any of a number of molecules within the cell of a living organism which can be used to identify the presence of the living organism in a sample. Although it is not a requirement, it is often a characteristic of the detection metabolite that it will disintegrate soon after the destruction of the cell. Thus, the detection of a detection metabolite often not only indicates the presence of the organism in the sample, it also determines whether the organism is still alive and active. This enhanced sensitivity to living organisms can be extremely useful, especially when the organism is only disease producing in its active form. Examples of detection metabolites include adenine nucleotides such as adenosine 5' monophosphate (AMP), adenosine 5' diphosphate (ADP) and adenosine 5' triphosphate (ATP). The adenine nucleotides are important in biolcgical energy metabolism. All living cells contain adenine nucleotides. The preferred adenine nucleotide and the preferred detection metabolite is ATP. Although ATP is the preferred detection metabolite for purposes of the present invention, other detection metabolites can be effectively utilized. (S. Yamashoji S., et al. (1989) Anal. Biochem, 181:149-152). Examples of other detection metabolites include $NAD^+$, NADH, $FMNH_2$, or FMN, which are all substrates for the bacterial luciferase enzyme (Wannlund, V. and M. DeLuca, 1983, Meth. Enzym., Vol. 92, pp. 426-432).

The term "light producing enzyme" is meant to include any enzyme which, upon catalyzing a reaction with a substrate, emits light as a by-product. An example of a light producing enzyme is luciferase.

The term "polyclonal antibodies" means antibodies produced in the normal immune response to an antigen consisting of a number of closely related but not identical proteins. The variation reflects the fact that they are formed by a number of lymphocytes.

The term "monoclonal antibody" describes an antibody produced by culturing a single type of cell.

The term "solid phase" or "solid carrier" is used herein to denote any support to which an antibody or cell-binding protein is bound or can be bound after antibody-cell reactions. The solid phase includes all variations of assay supports such as microtiter dishes, test tubes, polystyrene or plastic beads, resins, papers, plastics and magnetic particles. Additionally, the solid phase includes a dipstick, i.e., a stick or probe made of wood or another non-reactive material to which an antibody is bound. The dipstick is intended to be manually placed in a fluid sample which includes an antigen for reaction of the antibody with the antigen.

While various procedures utilized in the overall invention may be individually known to the art, the combination of these procedures and the advantages have not heretofore been disclosed or suggested. General methods known to the art, which play a part in the overall assay techniques of the present invention include the immobilization of cell binding proteins or antibodies on a solid phase, the binding of an antigenic cell substance to the cell binding protein using biotinylation reactions, standard immunologic and ELISA procedures, and assay procedures related to detection metabolite determinations using a light producing enzyme system. A discussion of one or more of the above-noted techniques can be found in the following references, which are incorporated herein by reference: Bacteriological Analytical Manual, 6th Ed., USDA, 1984; Marcel Dekker, Inc., New York; Leach, F.R. and J.J. Webster (1986) Methods in Enzymology, Vol.

133, pp. 22-27; Stanley, P.E. (1986), Methods in Enzymology, Vol. 133, pp. 14-22; O'Shannessy, D.J. and R.H. Quarles (1987), J. Immunological Methods, Vol. 99, pp. 153-161; Johnstone, A. and R. Thorpe (1982), Immunochemistry in Practice, Oxford, London; and Maniatis, T. et al. (1982) Molecular Cloning, Cold Spring Harbor.

The present invention is specifically directed to the use of cell capture techniques coupled with ATP detection as a method of cell detection. In this application, cells are captured onto a solid phase by any number of means, washed of any contaminating components or non-specific cells and lysed to release ATP for assay by the firefly-luciferase enzyme system. While it is within the scope of the present invention to utilize detection metabolites, cell binding molecules and light-emitting enzymes as described above, the description of the present invention will focus on the preferred ATP detection method utilizing an antibody-antigen ELISA procedure and the firefly-luciferase enzyme assay system.

Cell Capture Technique

The cell capture technique is conducted according to any of a number of well known enzyme immunoassay systems. Examples of some systems are described in Voller, A.D., et al. (1980), "Enzyme-linked Immunosorbant Assay," Manual of Clinical Immunology," 2nd ed., ed. N.R. Rose and H. Friedman. Am. Soc. for Microbiology, Washington, D.C., which is incorporated herein by reference for descriptions of the enzyme immunoassay systems. The immunoassay technique is based upon the formation of a complex between an antigenic cell substance in a fluid sample and à cell binding molecule, preferably an antibody or a system of antibodies, which permits the "capture" of the antigen onto a solid phase carrier or substrate. The antibodies may be either polyclonal or monoclonal antibodies. Any other macromolecular entity containing ATP which can be captured with an antibody is also a target and is covered in the invention.

The support system includes all solid phase carriers to which an antibody or capturing molecule can be coupled. This includes all matrices of any of a number of compositions and designs to which antibodies have been coupled, adsorbed or attached to by chemical or physical means. The attachment may be direct or may include bridging molecules such as biotin, avidin (streptavidin), secondary antibodies, lectins, protein A or G, and any other antibody binding protein or nucleic acid. The cells are captured onto the solid phase by any of a number of means known to the art.

Washing

The treated samples are washed one or several times with a suitable buffer solution, such as phosphate buffered saline, to rid the sample of any non-specific cells or other contaminating materials. It is within the scope of the present invention to use any buffer solution which will effectively wash the treated samples. Examples include phosphate buffered saline (Thorpe, R., supra.) (0.15M NaCl) which may contain a surfactant, or other buffers such as Tris (tris-hydroxymethylaminomethane) hydrochloride containing saline and a surfactant. In some instances washing may be effected by altering conditions of pH, ionic strength, or by including chaotrophic molecules.

Cell Lysis

Cell lysis is conducted to release the detection metabolite (ATP) for assay. Methods for extracting adenine nucleotides are referenced in Lundin and Thore, November 1975, Appl. Microbiol. vol. 30, No. 5, pp. 713-721 and Prioli and Brown, 1904, Anal. Appl. of Bioluminescence and Chemiluminesence, pp. 81-84, which are incorporated herein by reference for a description of nucleotide extraction procedures. Cell lysis is generally accomplished by the addition of a strong inorganic acid such as nitric acid, leaving a solution of free material, ATP and the acid. Other suitable acids include perchloric acid, trichloroacetic acid (TCA) and sulphuric acid or any inorganic acid that is highly ionized. The necessary concentration of the acid depends on the type of acid and the type of bacterial cell to be ruptured. Other physical or chemical means of releasing ATP from a captured cell may also be utilized. These include detergents, organic solvents, boiling aqueous solutions, or other membrane reactive molecules that may release ATP from bacterial cells.

Detection and/or Measurement

The detection and quantification of detection metabolites such as ATP can be accomplished using luminescence as an analytical tool (Sanville, C., June 1985, American Clinical Products Review). Luminescence has emerged as a leading technological advancement for the rapid, sensitive and simplified detection

and analysis of biological compounds such as ATP, NADH, FMN and other cell detection metabolites.

There are three key reactions on which many of the applications of luminescence are based:

1) ATP/firefly luciferase reaction.

$$ATP + Luciferin + Mg^{++} + O_2 \xrightarrow{FFL_*} Products + LIGHT$$

* FFL = Firefly luciferase

2) NAD(P)H/FMH$_2$ Bacterial luciferase reaction.

$$NAD(P)H + FMN + H^+ \xrightarrow{OXIDOREDUCTASE} FMNH_2 + NAD(P)^+$$

$$FMNH_2 + O_2 + RCHO \xrightarrow{BL_*} FMN + RCOOH + H_2O + LIGHT$$

* BL = bacterial luciferase

* RCHO = long chain aldehyde

3) Chemiluminescent reactions involving luminol.

$$Metal\ Ion + H_2O_2 + Luminol \longrightarrow H_2O + Light$$

Luminescence immunoassays can be performed using luminescent labels including luminol, isoluminol, acridium esters, trioxylate compounds and various luciferase enzymes.

As indicated previously, the preferred cell detection metabolite is ATP. Because it is the primary energy donor of living organisms, all living cells contain ATP. As a cell dies, it rapidly loses its stores of ATP. Thus, the measurement of ATP can be used as a means to determine cell viability.

ATP is most conveniently measured using the firefly-luciferase system. (Lundin, A., supra.; Lundin, A., Bioluminescence and Chemiluminescence, 1981, Academic Press, Inc. pp. 187-196; L. Kricka, et al. 1983, Anal. Biochem 129:392-397: A. Lundin, 1976, Anal. Biochem 175:611-620). Other luciferase systems such as click beetle luciferase can also be used. (Wood, K.V. et al, 1989, Science, 44, 700-702.

The light output from the reaction can be measured on simple instrumentation and can be quantitated. The light emission may be measured with luminometers using photomultipliers or diodes as light detectors or with photographic techniques, such as Polaroid cameras.

The sensitivity can be increased by concentrating the cells before the cell lysis process. Such conventional techniques include filtration or centrifugation.

Reference is now made to the figures for various embodiments of the present invention.

Referring now to Figure 1, the present invention is illustrated using the "ELISA" format. In this technique, a microtiter well or test tube is coated with an antibody. The antibody may either be polyclonal or monoclonal and must be specific to the cell type of interest, i.e., the target cell. The target cell, which is a component of a fluid sample, which may include non-cellular sample components and non-specific cells,

is then added to the microtiter well, where the target cell binds to the antibody. Unbound material is then washed away. This procedure is analogous to other referenced ELISA procedures. See, for example, D'Aoust, J. Y., et al. supra., 1986. The captured cells are then lysed or ruptured, by means of an acid such as nitric acid or detergents, to release the cellular ATP. The presence and/or concentration of the ATP in solution is then measured by the firefly-luciferase reaction system.

Referring now to Figure 2, the present invention is illustrated by utilizing a dipstick format. Rather than coating the microtiter well or test tube with the specific antibody, the antibody is bound to a dipstick, a sterile stick having on its surface antibodies designed to react with a target antigenic cell of interest within a fluid sample housed in a test tube. Reference is made to Herrman, J. E. (1981) Metho. Enz. vol. 73, pp. 239-244; and Pesce, A. J. (1977) Biophysica Acta, vol. 492, pp. 399-407, which are incorporated herein for a description of this process. The specific antigenic cell is then bound to the dipstick, which is washed to remove all non-cellular sample components. The dipstick is then placed into a lysis solution to lyse the cells and release the cellular ATP. The ATP assay test is then conducted using the firefly luciferase-luciferin system.

Reference is now made to Figure 3 for a third embodiment employing a magnetic solid phase. In this embodiment, the antibody is coated onto a magnetic particle which is added to a buffer solution. The fluid sample, containing the target cell, is then added to the test tube in order to bind specific cells to the antibody coated magnetic particle. A magnetic field is then applied to the wall of the test tube in order to attract the magnetic particles to the wall. The remaining solution is then aspirated from the test tube, leaving the magnetic particles attached to the wall of the test tube. Reference is made to Menz, E. T. (September 1986), American Biotechnology Laboratory; and Birkmeyer, R. C., et al. (1987), Clinical Chemistry, vol. 33, No. 9, pp. 1543-1547, which are incorporated herein by reference for a description of this process. The cells are lysed and ATP is released from the cells bound to the antibody coated magnetic particles. The detection of the presence and concentration of the ATP is then performed via the firefly-luciferase assay. Similarly, antibodies bound to the antigenic cell surface and the antibody-cell complexes can be captured on the magnetic particles. Extraction of ATP and detection would proceed as previously described.

Figure 4 illustrates another embodiment of the present invention utilizing a magnetic solid phase coated with avidin or streptavidin and a biotinylated cell-specific antibody. A fluid sample containing the target cells along with non-specific cells and non-cellular components is placed in a test tube. A biotinylated cell specific antibody is then added which binds to the target cell. The avidin or streptavidin coated magnetic particle is then bound to the biotinylated antibody forming a cell complex. The cell complex is then captured on the test tube wall via a magnet. The remaining fluid sample including the non-specific cell components and non-cellular components is aspirated leaving the magnetic particle complex magnetically bound to the test tube wall. Reference is made to Menz, E. T., supra., 1986; and Birkmeyer, R. C., et al., supra., 1987, which are incorporated herein by reference for a description of this process. ATP from the captured cells is released and is assayed by the firefly-luciferase technique. In like manner, the biotinylated antibody already bound to an avidin or streptavidin magnetic particle can be incubated with the sample in a single step procedure.

Reference is now made to Figure 5 which illustrates yet another embodiment of the present invention utilizing a magnetic solid phase with protein A. In this embodiment, a fluid sample containing the target cells, non-specific cells and non-cellular material is placed in a test tube. A target cell specific antibody is then bound to the target cells. The antibody is then bound to the protein A magnetic particles forming a target cell complex. The cell complex is then retained on the test tube wall via a magnet in a similar fashion as that disclosed with respect to the embodiment illustrated in Figure 4. The fluid sample solution is then aspirated leaving the magnetic particle target cell complex bound to the test tube wall. Reference is made to Menz, E. T., supra., 1986, which is incorporated herein by reference for a description of this process. ATP from the captured target cell is then released for assay with the firefly-luciferase technique.

Thus, the present invention describes the use of specific cell capture techniques coupled with ATP detection as a method of target cell detection. In these applications, target cells are captured onto a solid phase by any number of means, washed of any contaminating components or non-specific cells, and cellular ATP is released for assay by the firefly-luciferase enzyme system.

The present invention is also directed to kits that utilize this method.

A diagnostic kit for the detection of the presence of an antigenic cell substance in a sample would include the following:

a) a container containing a cell binding molecule against the antigenic cell substance coupled to a water insoluble solid carrier;

b) a container containing a buffer solution;

c) a container containing a cell lysing or detection metabolite releasing solution; and

d) a container containing assay reagents to perform an assay for the detection metabolite.

The kit can be conveniently modified to accommodate solid carriers, cell binding molecules, cell lysing solution and assay reagents described hereinabove and in the following examples.

## DESCRIPTION OF THE PREFERRED EMBODIMENTS

The present invention is preferably directed to the detection of contaminating organisms in a sample, such as a food or fluid sample. For example, a food specimen thought to be contaminated with Salmonella cells is enriched for Salmonella cells by growth in an enrichment broth and added to a microtiter dish well that has been previously coated with antibodies to Salmonella cells. Reference is made to D'Aoust, et al. (supra.) for a description of a suitable enrichment technique. The Salmonella cells are allowed to bind to the antibodies and the plate is washed to remove non-specific cells or other materials. A lysis solution containing acid and/or detergent is then added to the well to lyse the bacterial cells. An aliquot of cell lysis solution is then added to a firefly-luciferase reagent system whereby the amount of ATP in the sample is very accurately measured. The amount of ATP is then used to estimate the number of Salmonella cells in the original sample. A kit for this purpose would contain an antibody coated solid phase or a variation, buffers, wash solutions, a cell lysing solution and ATP assay reagents to perform the assay.

The invention is further illustrated by the following examples.

### Example 1

Example 1 was designed to disclose the capture and detection of Salmonella cells. A flat-bottomed ELISA plate (Nunc ™, Naperville, Ill) was coated with anti-Salmonella antibody (Kirkegard and Perry, Bac Trace™, Catalog No. 01-91-99, Gathersburg, Md). One milligram of lyophilized antibody was reconstituted with 50% (v/v) glycerol. This glycerol solution was diluted 1:100 (22 uL to 2.2 mL) in 50 mM sodium carbonate, pH. 9.6. Fifty uL of the sodium carbonate diluted antibody was placed into each well of the microtiter dish and antibody was allowed to bind overnite (15 hours) at room temperature (23 °C). A row of wells on the plate was not coated with antibody to serve as a minus antibody control. The next morning the antibody solutions were discarded (by flicking out the liquid) and the wells were rinsed three times each with phosphate buffered saline (PBS). Each well was then blocked with 100 uL of a solution of PBS containing 1% (w/v) casein hydrolysate (Sigma, St. Louis, MO) and 5% (v/v) Tween-20 (Bio-Rad Laboratories, Richmond, CA) for 1 hour at 23 °C. Following blocking, the plate was washed three times with PBS containing 0.05% Tween-20.

Two overnight cultures of bacterial cells were prepared for use in the capture assay. The first was Salmonella typhimurium (American Type Culture Collection, ATCC 14028, Rockville, Md) which is the capture specific, and Klebsiella pneumoniae (ATCC 23357) which is a control, non-specific organism. Both cultures were grown overnight in flasks containing LB broth at 37 °C at 250 rpm. Cells for each culture were serially diluted in PBS ($10^{-1}$, $10^{-2}$, $10^{-3}$, $10^{-4}$, $10^{-5}$, $10^{-6}$, $10^{-7}$). Fifty uL of the dilutions were added to appropriate microtiter wells as shown in Table 1.

## TABLE 1

### ELISA CAPTURE ASSAY DESIGN: Salmonella

| ROW | COLUMN 1 | 2 | 3 | 4 | 5 | 6 | 7 |
|---|---|---|---|---|---|---|---|
| A[1] | $S^{-1}$ | $S^{-2}$ | $S^{-3}$ | $S^{-4}$ | $S^{-5}$ | $S^{-6}$ | $S^{-7}$ |
| B[2] | $S^{-1}$ | $S^{-2}$ | $S^{-3}$ | $S^{-4}$ | $S^{-5}$ | $S^{-6}$ | $S^{-7}$ |
| C[2] | $K^{-1}$ | $K^{-2}$ | $K^{-3}$ | $K^{-4}$ | $K^{-5}$ | $S^{-6}$ | $S^{-7}$ |

$S^{-1}$ is Salmonella cells at $10^{-1}$ dilution
$K^{-1}$ is Klebsiella cells at $1-^{-1}$ dilution

[1] Row A has no antibody.

[2] Rows B and C were coated with anti-Salmonella antibody.

The cells were allowed to incubate for 1.5 hours at 23° C. In addition, 50 uL of each of the cell dilutions was plated on LB agar (Maniatis, T. et al., supra.) in order to determine the number of cells in the original overnight cultures.

Following the cell incubation, the wells were individually aspirated into a sodium hypochlorite trap via vacuum, and each well was washed three times with 300 mL PBS, aspirating the PBS after each wash step.

After washing the wells, 50 uL of a solution containing 2% (w/v) TCA, 2mM ethylenediaminetetraacetic acid (EDTA) and 0.01% xylenol blue was added to each well to lyse any bacteria present. The plate was then incubated for 30 minutes at room temperature (23° C).

Five uL of the TCA solutions from each of the wells was placed in luminometer tubes (Evergreen Scientific, Los Angeles, CA, Cat. 214-2301-030) and neutralized with 0.135 mL 0.1 M Tris-acetate, pH 7.75.

Finally, 20 uL of a luciferase-luciferin assay reagent for ATP (Promega Corp. Madison, WI) was added to each cuvette, vortexed and placed into the light chamber of a Turner Model TD-20e luminometer (Turner Designs, Inc., Mountain View, CA). After a 5 second delay, light output was integrated over a 10 second interval. Results for each sample are recorded in Relative Light Units (RLU).

The results from this experiment are summarized in Table 2.

TABLE 2

ELISA CAPTURE RESULTS: Salmonella RLU

| ROW | COLUMN 1 | 2 | 3 | 4 | 5 | 6 | 7 |
|---|---|---|---|---|---|---|---|
| A | 2.05 | 0.33 | 0.39 | 0.21 | -- | -- | -- |
| B | 89.9 | 30.3 | 13.0 | 0.68 | 0.24 | 0.20 | 0.22 |
| C | -- | 0.45 | 0.41 | 0.17 | 0.12 | -- | -- |

Colony counting results from the LB agar gave a cell number in the original Salmonella overnight of 3.4 x $10^9$ cells per mL and 2.1 x $10^9$ cells per mL for the Klebsiella overnight culture. Using the numbers of colonies formed in the plating of the cell dilutions, Figure 6 illustrates a summary of the experimental results.

In the presence of anti-Salmonella antibody on the ELISA plate, increasing Salmonella cells are captured as the number of input cells are increased. In the absence of Salmonella antibody, very low (less than 1 RLU) numbers of cells are captured until greater than $10^8$ cells are added to the well. Even at this loading level, only about 2 RLU of light is observed; this is very likely due to non-specific adsorption of cells to the ELISA well.

A non-Salmonella cell, Klebsiella, shows a very low capture efficiency in this experiment. Even at $10^7$ cells, less than 1 RLU is observed. This demonstrates the specificity of the proposed invention.

The sensitivity of this experiment is on the order of 7 x $10^4$ Salmonella cells (twice the signal of the 0.2 RLU background). This sensitivity can likely be improved upon, by addition of a larger portion of the cell lysis solution to the light reaction. Only one-tenth of the lysate was added in this example.

Example 2

Example 2 was designed to disclose the capture and detection of the yeast Candida albicans using the present invention. IgG from antiserum to Candida albicans (Difco, Detroit, MI) was purified on a Protein A sepharose CL-4B (Pharmacia, Piscataway, NJ) column. Approximately 2.5 mL of antiserum in 0.2M sodium phosphate, pH 8.2 was applied to a 3 mL column previously equilibrated with 10 mM sodium phosphate, 0.15 M NaCl, pH 8.2 and washed with 10 column volumes of buffer. IgG was eluted from the column in 2 fractions (6 mL total) with 0.1M sodium citrate, pH 3.0. The eluted IgG (2 mg) was dialyzed against PBS and aliquots were stored frozen at -70° C.

IgG was diluted 1:20 in 50 mM sodium carbonate, pH 9.6, and 50 uL was placed into flat-bottom microtiter dish (Nunc™, Naperville, Ill) wells and allowed to adsorb overnight at 23° C. In the morning wells were rinsed and aspirated three times with 300 mL PBS containing 0.05% Tween-20. These wells and another row of wells (no IgG bound) were blocked with PBS containing 1% casein hydrolysate and 5% Tween-20 for 1.5 hours at 23° C. All wells were then rinsed three times with PBS containing 0.05% Tween-20.

Candida albicans cells were grown in flasks containing LB broth overnight at 37° C at 250 rpm shaking. Cells were serially diluted in PBS ($10^{-1}$, $10^{-2}$, $10^{-3}$, $10^{-4}$, $10^{-5}$, $10^{-6}$, $10^{-7}$) and 50 uL of each dilution and undiluted overnight controls were incubated and then plated on LB agar to determine cell numbers in the original overnight culture. Cells (50 uL of each dilution) were added to microtiter dish wells as shown in Table 3.

TABLE 3

ELISA CAPTURE ASSAY DESIGN: Candida

| ROW | COLUMN 1 | 2 | 3 | 4 | 5 | 6 | 7 | 8 |
|---|---|---|---|---|---|---|---|---|
| A[1] | $C^{-0}$ | $C^{-1}$ | $C^{-2}$ | $C^{-3}$ | $C^{-4}$ | $C^{-5}$ | $C^{-6}$ | $C^{-7}$ |
| B[2] | $C^{-0}$ | $C^{-1}$ | $C^{-2}$ | $C^{-3}$ | $C^{-4}$ | $C^{-5}$ | $C^{-6}$ | $C^{-7}$ |

[1] Row A has no antibody

[2] Row B was coated with antibody to Candida

The cells were allowed to incubate for 1.5 hours and then cells were removed by aspiration and the wells were rinsed three times with 300 uL PBS.

To each well a solution containing 8% TCA, 8 mM EDTA, and 0.02% xylenol blue was added and allowed to incubate for 20 minutes at room temperature. For the light reaction, 5 uL of the TCA, EDTA, xylenol blue solution was neutralized in a luminometer cuvette with 135 uL of 0.1 M Tris-acetate, pH 7.75. Light output was determined by the addition of 20 uL of luciferase-luciferin reagent and measured as described in Example 1.

RESULTS AND DISCUSSION:

Colony counting results from LB agar plates gave a cell number for the overnight culture of 9.2 x $10^7$ cells per mL. Results from the capture assay are summarized in Table 4 following and illustrated in Figure 7.

TABLE 4

ELISA CAPTURE RESULTS: Candida RLU

| ROW | COLUMN 1 | 2 | 3 | 4 | 5 | 6 | 7 | 8 |
|---|---|---|---|---|---|---|---|---|
| A | 2.78 | 0.98 | 0.16 | 0.13 | 0.10 | 0.11 | 0.15 | 0.11 |
| B | 227.5 | 143.4 | 22.7 | 2.19 | 0.65 | 0.23 | 0.22 | 0.16 |

Figure 7 illustrates a dose response of increasing RLU for increasing cells added when anti-Candida antibody is coated on the ELISA plate wells. In the absence of antibody the capture (RLU) is significantly less (about one hundred fold). The detection limit (at about 2 times background) is approximately 200 cells.

It is understood that the invention is not confined to the particular constructions and arrangements herein illustrated and described, but embraces such modified forms thereof as come within the scope of the following claims.

## Claims

1. A process for determining the presence of an antigenic cell substance including a detection metabolite

in a sample, comprising:

a) contacting the sample with cell binding molecules designed to bind to the antigenic cell substance, the cell binding molecules being bound to a solid carrier insoluble in the sample, to form a complex between the cell binding molecules and antigenic cell substance;
b) separating the solid carrier from the sample;
c) lysing the cell wall of the antigenic cell substance to release the detection metabolite;
d) adding a light producing enzyme reagent to the treated sample of step c); and
e) detecting the presence of the detection metabolite in the treated sample of step d).

**2.** A process according to claim 1 wherein the detection metabolite is ATP.

**3.** A process according to claim 1 wherein the detection metabolie is $NAD^+$, NADH, $FMNH_2$ or FMN.

**4.** A process according to any one of claims 1 to 3 wherein the light-producing enzyme is a firefly-luciferase reagent or click beetle luciferase reagent or a variation thereof.

**5.** A process according to claim 4 wherein the light producing enzyme is a bacterial firefly-luciferase reagent.

**6.** A process according to any one of claims 1 to 5 wherein the sample is a food, liquid, body fluid or a solution derived from a food, liquid or body fluid.

**7.** A process according to any one of the preceding claims wherein the antigenic cell substance is a prokaryotic cellular organism or an eukaryotic cellular organism.

**8.** A process according to any one of the preceding claims wherein the cell binding molecules are antibodies.

**9.** A process according to claim 8 wherein the antibodies are polyclonal antibodies or monoclonal antibodies.

**10.** A process according to any one of the preceding claims wherein the solid carrier is a microtiter dish, test tube, polystyrene beads, plastic beads, a resin, paper, a plastics material or a magnetic particle.

**11.** A process according to any one of claims 1 to 10 wherein the solid carrier is an ELISA plate or ELISA test tube.

**12.** A process according to claim 10 wherein the solid carrier is a magnetic particle coated with avidin or a magnetic particle coated with streptavidin and the antibodies are cell specific antibodies labeled with biotin or iminobiotin.

**13.** A process according to claim 10 wherein the solid carrier is a magnetic particle coated with protein A.

**14.** A process according to any one of claims 1 to 9 wherein the solid carrier is a dipstick coated with the antibodies.

**15.** A process according to any one of claims 1 to 14 werehin the cell binding proteins are directly attched to the solid carrier.

**16.** A process according to any one of claims 1 to 14 wherein the cell binding proteins are attached to the solid carrier by chemical means or by bridging molecules which are biotin, avidin, streptavidin, secondary antibodies, lectins, protein A or protein G.

**17.** A process according to any one of the preceding claims wherein the unreacted antigen is separated from the solid carrier by washing the solid carrier with a buffer solution.

**18.** A process according to claim 17 wherein the buffer solution is phosphate buffered saline.

**19.** A process according to any one of the preceding claims wherein the cell walls are lysed by the addition of an acid.

**20.** A process according to claim 19 wherein the acid is nitric acid, trichloroacetic acid, perchloric acid or sulphuric acid.

**21.** A process according to any of claims 1 to 18 wherein the cell walls are lysed by the addition of an organic solvent.

**22.** A process according to any one of claims 1 to 18 wherein the cell walls are lysed by the addition of a detergent or surfactant.

**23.** A process according to any one of the preceding claims wherein the concentration of the antigenic cell substance is the sample is determined wherein the emitted light is measured in step e) and which comprises:
f) calculating the concentration of the detection metabolite from the measurement or emitted light; and
g) calculating the antigenic cell concentration in the sample.

**24.** A process according to claim 23 for determining the presence and concentration of Salmonella in a sample.

**25.** A diagnostic kit for the detection of the presence of an antigenic cell substance in a sample, comprising:
a) a container containing a cell binding molecule against the antigenic cell substance coupled to a water insoluble solid carrier;
b) a container a buffer solution;
c) a container containing a cell lysing or detection metabolite releasing solution; and
d) a container containing assay reagents to perform an assay for the detection metabolite.

**26.** A kit according to claim 25 which has one or more of the features of claims 2 to 5, 7 to 16, 18 to 20 and 24.

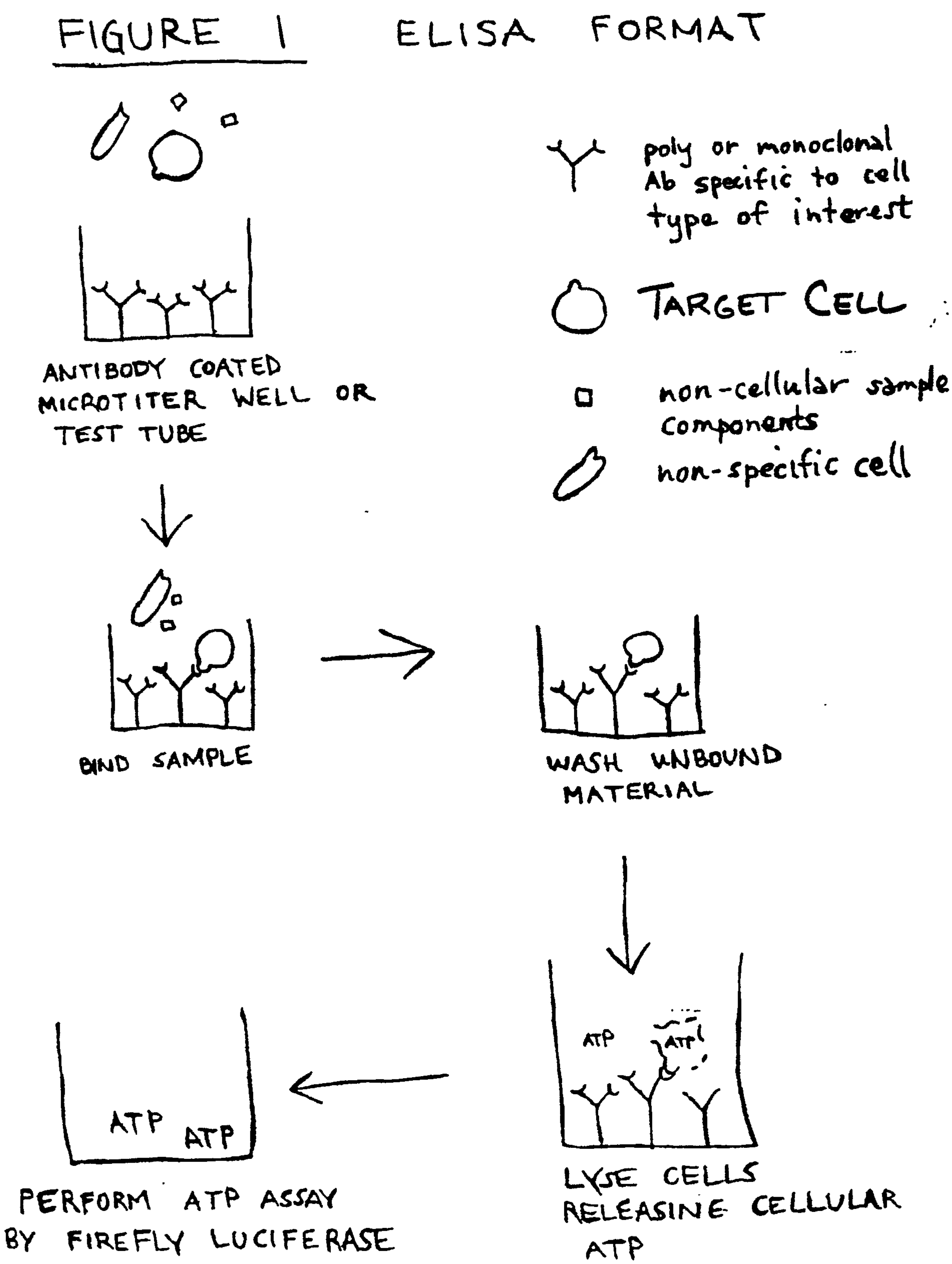
FIGURE 1 ELISA FORMAT
poly or monoclonal Ab specific to cell type of interest
TARGET CELL
non-cellular sample components
non-specific cell
ANTIBODY COATED MICROTITER WELL OR TEST TUBE
BIND SAMPLE
WASH UNBOUND MATERIAL
ATP
ATP
LYSE CELLS RELEASINE CELLULAR ATP
ATP ATP
PERFORM ATP ASSAY BY FIREFLY LUCIFERASE

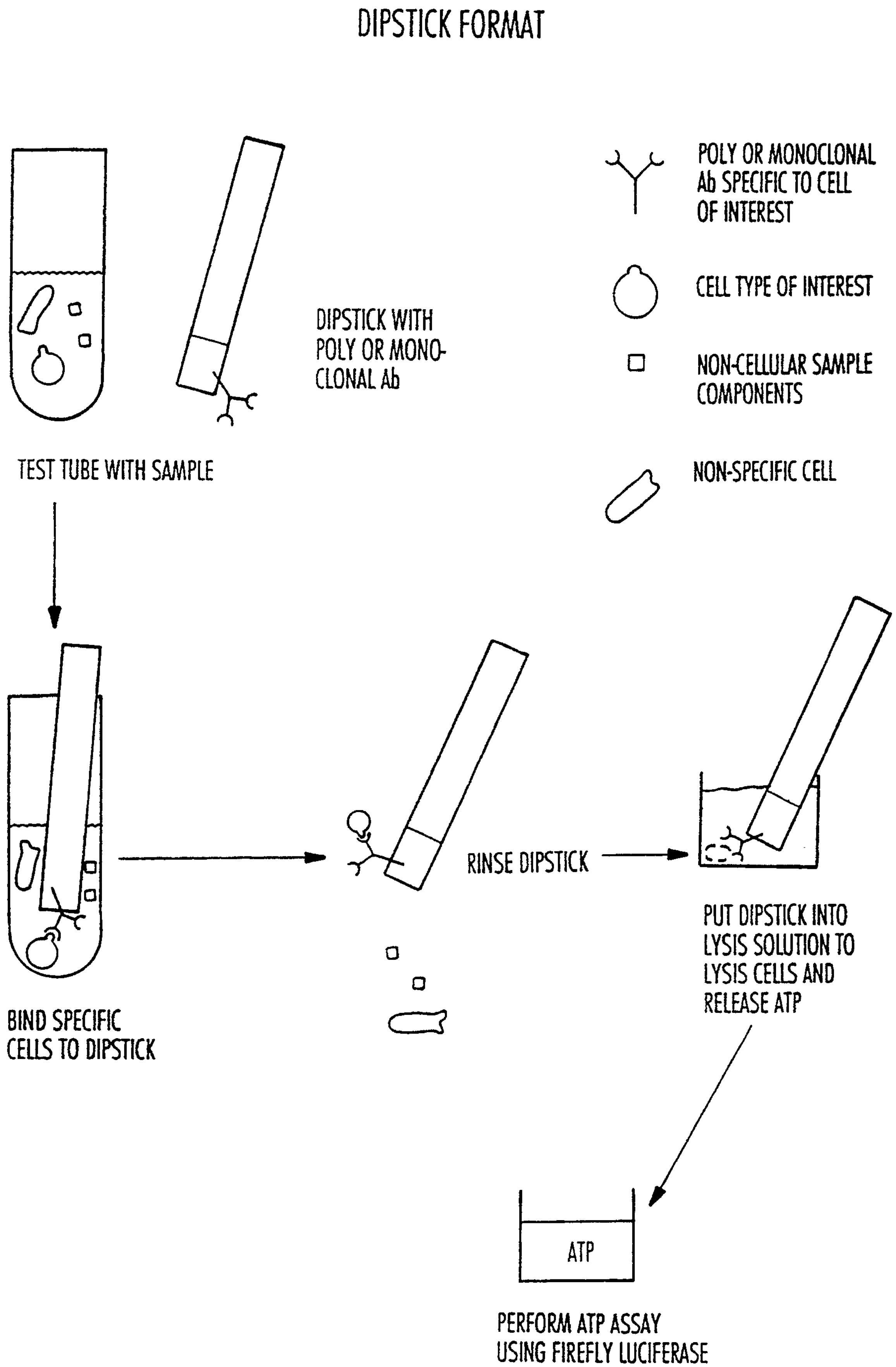
DIPSTICK FORMAT
POLY OR MONOCLONAL Ab SPECIFIC TO CELL OF INTEREST
CELL TYPE OF INTEREST
NON-CELLULAR SAMPLE COMPONENTS
NON-SPECIFIC CELL
DIPSTICK WITH POLY OR MONO-CLONAL Ab
TEST TUBE WITH SAMPLE
BIND SPECIFIC CELLS TO DIPSTICK
RINSE DIPSTICK
PUT DIPSTICK INTO LYSIS SOLUTION TO LYSIS CELLS AND RELEASE ATP
ATP
PERFORM ATP ASSAY USING FIREFLY LUCIFERASE

FIG. 2

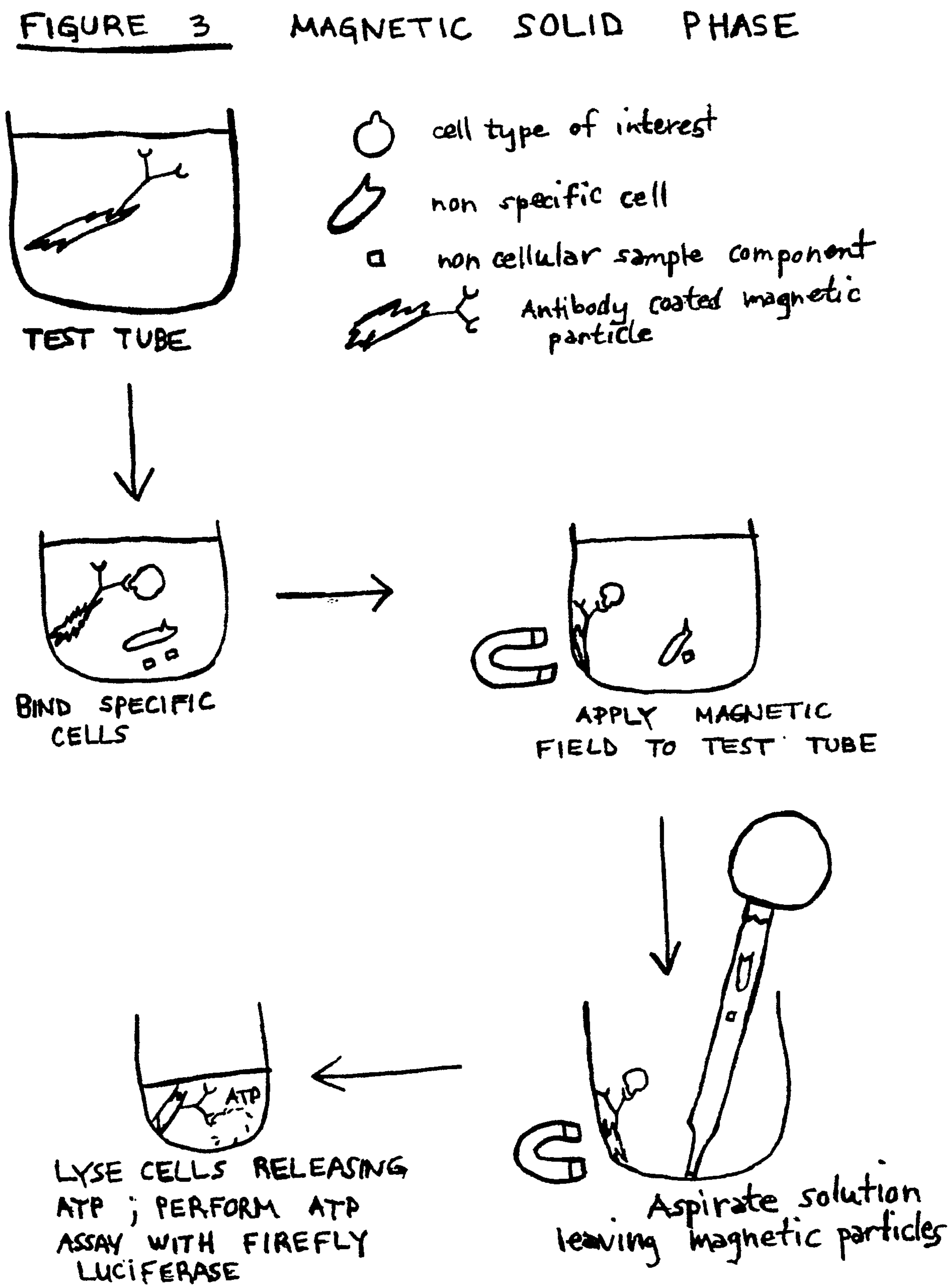
FIGURE 3 MAGNETIC SOLID PHASE
cell type of interest
non specific cell
non cellular sample component
Antibody coated magnetic particle
TEST TUBE
BIND SPECIFIC CELLS
APPLY MAGNETIC FIELD TO TEST TUBE
Aspirate solution leaving magnetic particles
ATP
LYSE CELLS RELEASING ATP ; PERFORM ATP ASSAY WITH FIREFLY LUCIFERASE

FIGURE 4 MAGNETIC SOLID PHASE USING BIOTIN/STREPTAVIDIN

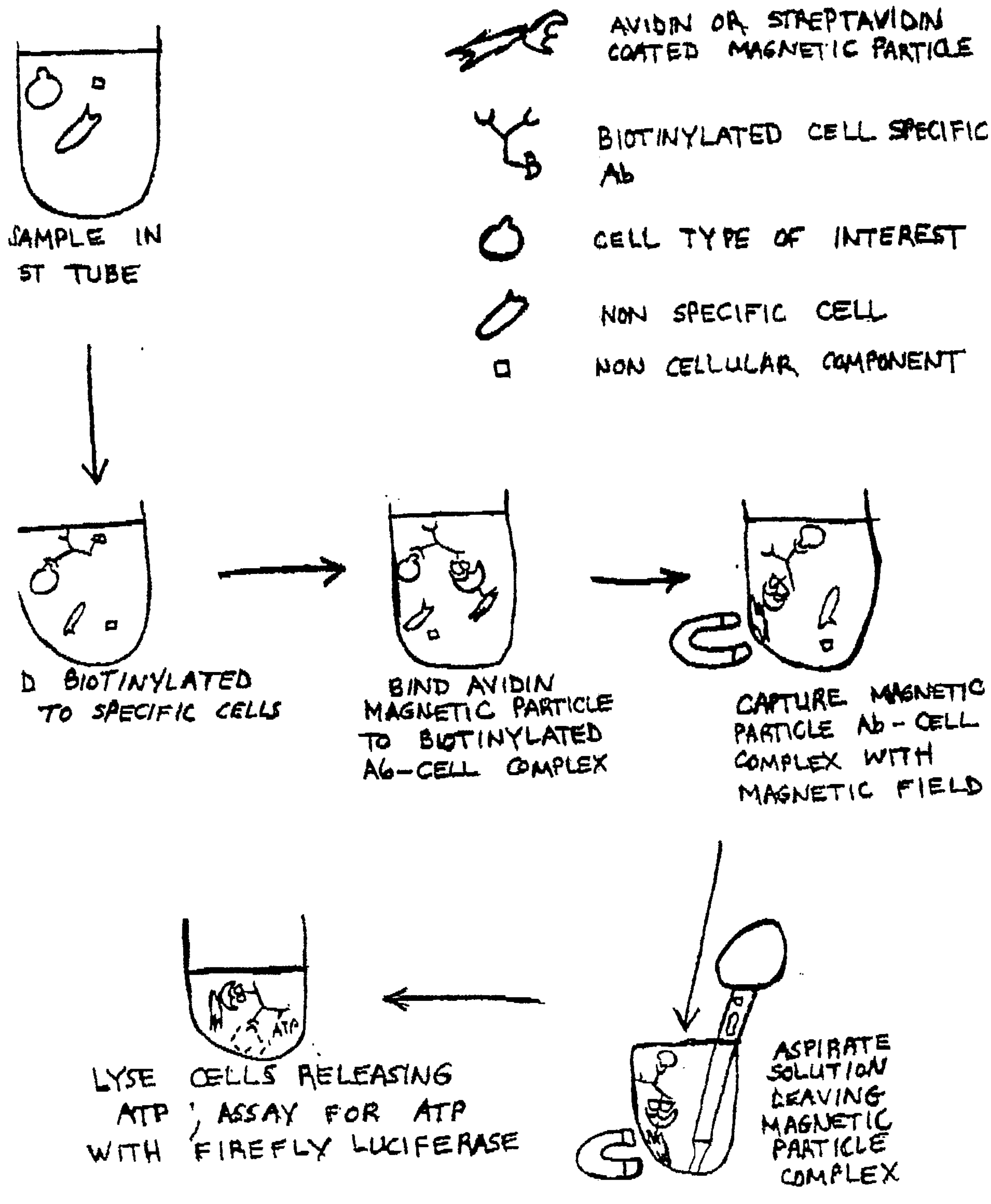

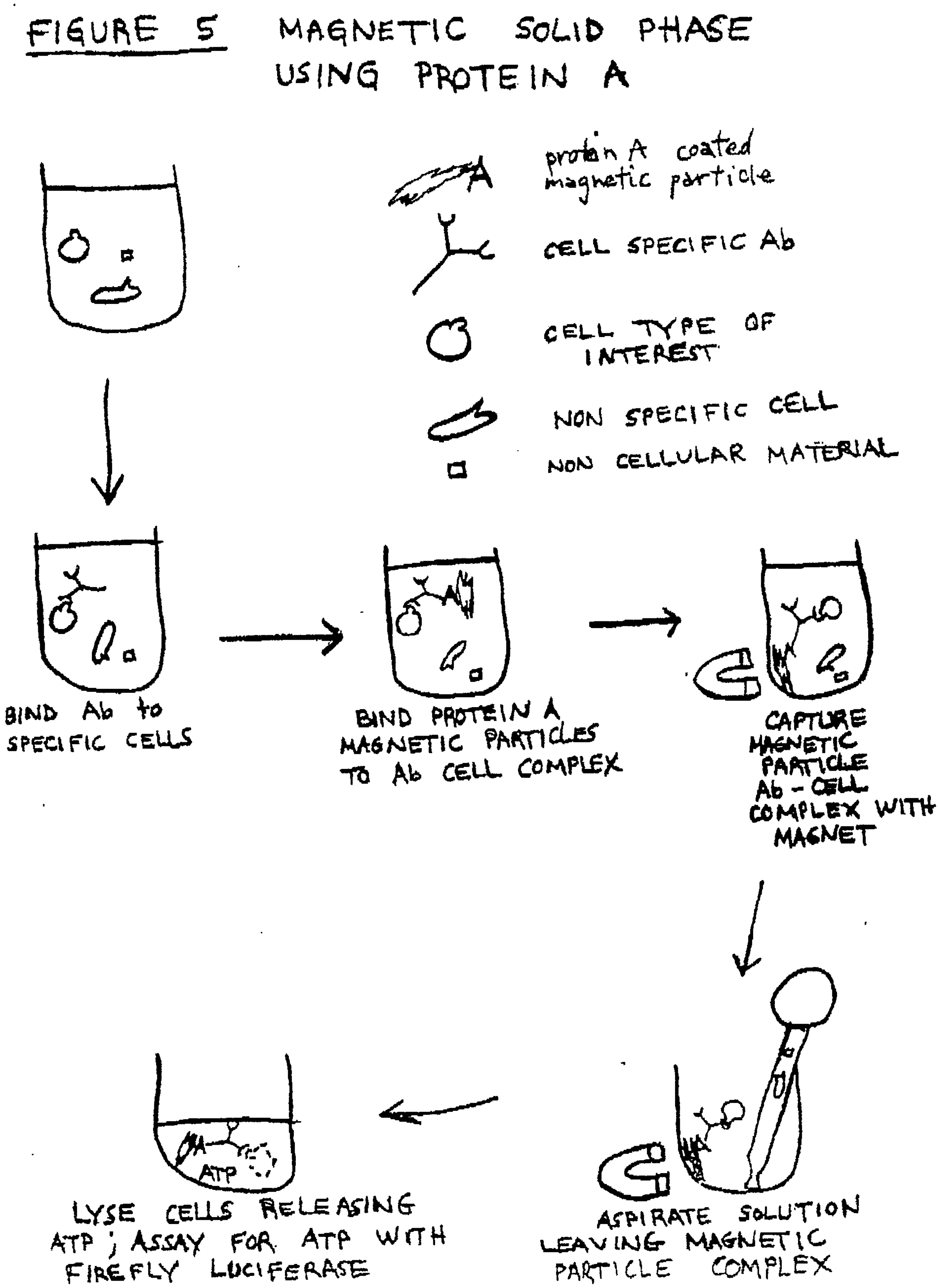
FIGURE 5 MAGNETIC SOLID PHASE USING PROTEIN A
protein A coated magnetic particle
CELL SPECIFIC Ab
CELL TYPE OF INTEREST
NON SPECIFIC CELL
NON CELLULAR MATERIAL
BIND Ab to SPECIFIC CELLS
BIND PROTEIN A MAGNETIC PARTICLES TO Ab CELL COMPLEX
CAPTURE MAGNETIC PARTICLE Ab - CELL COMPLEX WITH MAGNET
ATP
ASPIRATE SOLUTION LEAVING MAGNETIC PARTICLE COMPLEX
LYSE CELLS RELEASING ATP ; ASSAY FOR ATP WITH FIREFLY LUCIFERASE

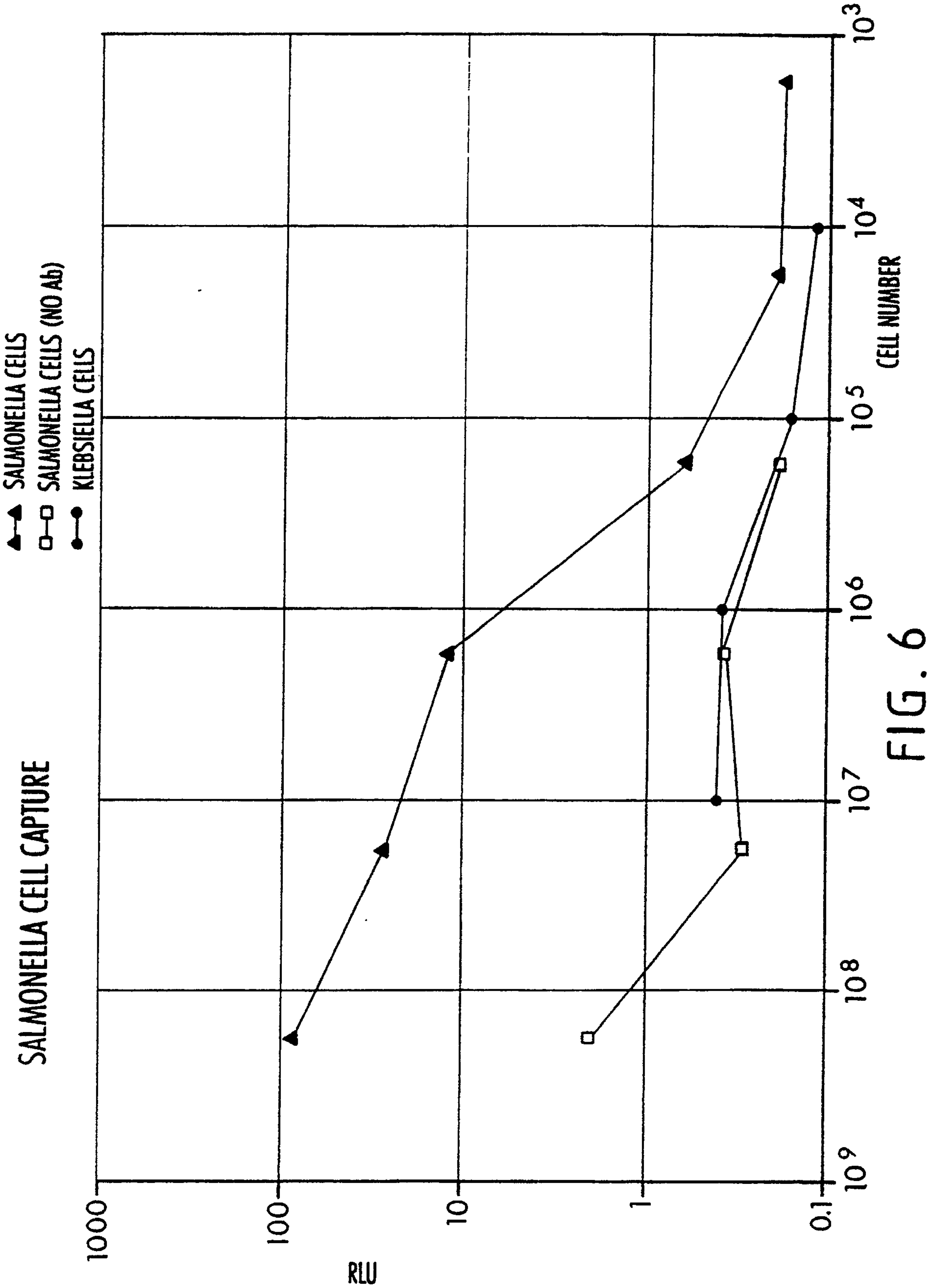
SALMONELLA CELL CAPTURE
SALMONELLA CELLS
SALMONELLA CELLS (NO Ab)
KLEBSIELLA CELLS
RLU
1000
100
10
1
0.1
CELL NUMBER
$10^9$
$10^8$
$10^7$
$10^6$
$10^5$
$10^4$
$10^3$

FIG. 6

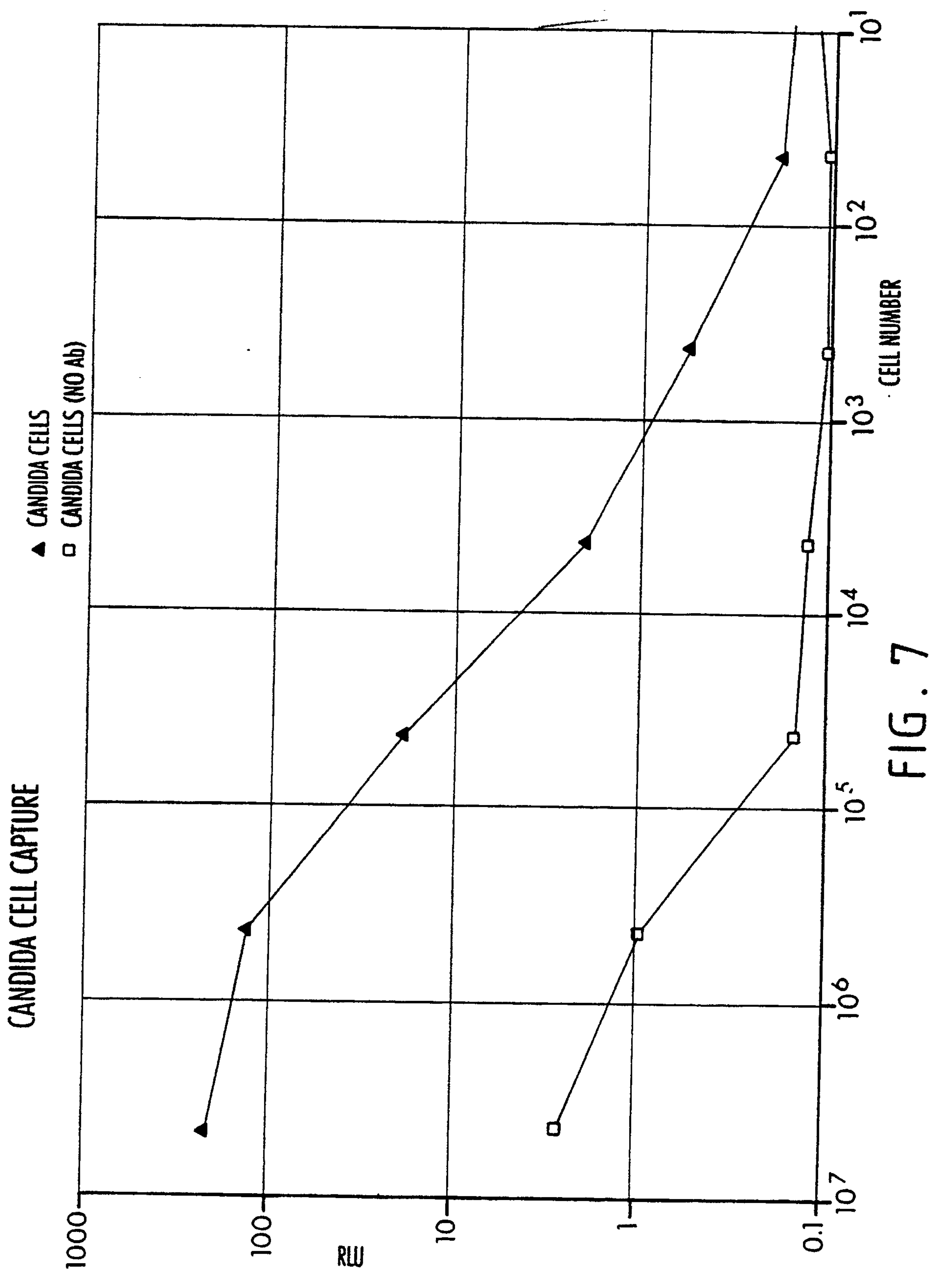
CANDIDA CELL CAPTURE
▲ CANDIDA CELLS
□ CANDIDA CELLS (NO Ab)
RLU
1000
100
10
1
0.1
$10^7$
$10^6$
$10^5$
$10^4$
$10^3$
$10^2$
$10^1$
CELL NUMBER

FIG. 7

European Patent Office

# EUROPEAN SEARCH REPORT

Application Number

**EP 91 30 0007**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | DE-A-3 224 484 (YEDA RESEARCH AND DEVELOPMENT CO., LTD, REHOVOT, IL) * Page 4, examples 24,25; figures 1-3 * | 1,2,25 | G 01 N 33/543<br>C 12 Q 1/66 |
| Y,D | US-A-3 745 090 (E.W. CHAPPELLE et al.) * The entire document * | 1,2,25 | |
| A,D | | 4-7, 19-22,24 | |
| A,D | WO-A-8 902 929 (LIFESCIENCE INTERNATIONAL AB) * The entire document * | 1,2,4-6, 23,25,26 | |
| A | US-A-3 660 240 (E.W. CHAPPELLE et al.) * The entire document * | 1,3-7,19, 20,23-26 | |
| A | EP-A-0 363 510 (BIOTEST AG) * Pages 2-3; examples 2,3 * | 1,6-11, 15-18,25, 26 | |
| A | EP-A-0 016 552 (NORTHWESTERN UNIVERSITY) * The entire document * | 1,6-18, 25,26 | **TECHNICAL FIELDS SEARCHED (Int. Cl.5)** |
| A,D | US-A-4 385 113 (E.W. CHAPPELLE et al.) * Abstract; claims 10-21 * | 1,2,4-6, 19,20,25, 26 | G 01 N<br>C 12 Q |
| A | WO-A-8 607 094 (UNIVERSITY OF WALES, COLLEGE OF MEDICINE, CARDIFF) * Abstract; claims * | 1,2,22 | |
| A | US-A-4 282 287 (R.W. GIESE) * The entire document * | 1,8-12,16 | |

-/-

The present search report has been drawn up for all claims

| Place of search | Date of completion of search | Examiner |
|---|---|---|
| The Hague | 29 May 91 | DOEPFER K-P. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention
E: earlier patent document, but published on, or after the filing date
D: document cited in the application
L: document cited for other reasons
&: member of the same patent family, corresponding document

European
Patent Office

# EUROPEAN SEARCH REPORT

Application Number

**EP 91 30 0007**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 153 283 (SYN-TEK AB)<br>* Abstract *<br>- - - - - | 1,6-12 | |
| | | | TECHNICAL FIELDS SEARCHED (Int. Cl.5) |

The present search report has been drawn up for all claims

| Place of search | Date of completion of search | Examiner |
|---|---|---|
| The Hague | 29 May 91 | DOEPFER K-P. |

CATEGORY OF CITED DOCUMENTS
- X: particularly relevant if taken alone
- Y: particularly relevant if combined with another document of the same catagory
- A: technological background
- O: non-written disclosure
- P: intermediate document
- T: theory or principle underlying the invention
- E: earlier patent document, but published on, or after the filing date
- D: document cited in the application
- L: document cited for other reasons
- &: member of the same patent family, corresponding document